(19) Europäisches Patentamt
European Patent Office
Office européen des brevets

(11) EP 1 685 120 B1

(12) **FASCICULE DE BREVET EUROPEEN**

(45) Date de publication et mention de la délivrance du brevet:
**17.03.2010 Bulletin 2010/11**

(21) Numéro de dépôt: **04805464.7**

(22) Date de dépôt: **16.11.2004**

(51) Int Cl.:
***C07D 307/91*** *(2006.01)* ***C07C 59/52*** *(2006.01)*
***C07H 15/203*** *(2006.01)* ***C07H 17/04*** *(2006.01)*

(86) Numéro de dépôt international:
**PCT/FR2004/002927**

(87) Numéro de publication internationale:
**WO 2005/049598 (02.06.2005 Gazette 2005/22)**

(54) **PREPARATIONS COLORANTES HYDROSOLUBLES JAUNES DERIVEES DES DIHYDROCHALCONES**

WASSERLÖSLICHE, GELBE, VON DIHYDROCHALKONEN GEWONNENE FÄRBEZUBEREITUNG

COLOURING HYDROSOLUBLE YELLOW PREPARATION DERIVED FROM DIHYDROCHALCONES

(84) Etats contractants désignés:
**AT BE BG CH CY CZ DE DK EE ES FI FR GB GR HU IE IS IT LI LU MC NL PL PT RO SE SI SK TR**

(30) Priorité: **17.11.2003 FR 0313414**

(43) Date de publication de la demande:
**02.08.2006 Bulletin 2006/31**

(73) Titulaires:
- **INSTITUT NATIONAL DE LA RECHERCHE AGRONOMIQUE**
  **75338 Paris (FR)**
- **Societe Cooperative Agricole Elle et Vire**
  **50890 Conde sur Vire (FR)**

(72) Inventeurs:
- **SANONER, Philippe**
  **F-50410 Percy (FR)**
- **GUYOT, Sylvain**
  **35630 La Chapelle-Chaussée (FR)**
- **LEGUERNEVE, Christine**
  **F-34070 Montpellier (FR)**
- **LEQUERE, Jean-Michel**
  **F-35590 Clayes (FR)**
- **DRILLEAU, Jean-François**
  **F-35000 Rennes (FR)**
- **RENARD, Catherine**
  **F-35132 Vezin Le Coquet (FR)**

(74) Mandataire: **Grosset-Fournier, Chantal Catherine**
**Grosset-Fournier & Demachy**
**54, Rue Saint-Lazare**
**75009 Paris (FR)**

(56) Documents cités:
**EP-A- 0 773 226** **EP-A- 0 850 948**
**FR-A- 2 822 466**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 685 120 B1

## Description

La présente invention a pour objet de nouvelles préparations colorantes hydrosolubles jaunes dérivées des dihydrochalcones, leur procédé de préparation, et leurs utilisations, notamment dans des compositions alimentaires, pharmaceutiques, ou cosmétiques.

L'industrie agroalimentaire et cosmétique dispose de peu de solution en matière de pigments jaunes fortement hydrosolubles. Les pigments les plus utilisés sont le plus souvent la tartrazine (E102) et la curcumine (E100). La tartrazine résulte d'une synthèse chimique et présente un certain pouvoir allergène. Elle est actuellement interdite en Autriche et en Norvège. La curcumine est, quant à elle, faiblement hydrosoluble.

La phloridzine est un polyphénol naturel et spécifique de la pomme, dont l'oxydation enzymatique a déjà fait l'objet de travaux publiés (Raa et Overeem, 1968 ; Oszmianski et Lee, 1991, Ridgway *et al.*, 1997), sans toutefois que ces travaux aient abouti ni à l'isolement ni à l'élucidation de la structure chimique des pigments formés.

En outre, la phloridzine est également connue pour son activité antioxydante (Miller *et al.*, 1998).

Un des principaux buts de la présente invention est de fournir un nouveau composé utilisable en tant que colorant jaune hydrosoluble alimentaire, et présentant l'avantage :

- de ne pas être issu de la synthèse chimique, mais dérivé de la phloridzine par un procédé enzymatique et correspondant à une fraction de la coloration naturelle d'un jus de pomme,
- de ne pas être cytotoxique (tests *in vitro*)
- d'être librement soluble dans l'eau, et donc facilement lavable à l'eau,
- de présenter une grande capacité de coloration,
- d'apporter une couleur jaune stable pour un pH inférieur à 5-6, ce qui correspond à la plupart des produits agro-alimentaires, et orange pour les pH supérieurs,
- de permettre de conserver une grande luminosité au produit à colorer,
- de permettre d'obtenir des couleurs vertes (type sirop de menthe verte) en mélange avec des colorants bleus.

L'invention a également pour but de fournir des compositions alimentaires contenant un tel colorant, ainsi que des compositions pharmaceutiques et cosmétiques contenant à titre de principe actif ce composé et/ou des composés précurseurs de ce colorant, présentant un intérêt pour leur activité antioxydante ou pigmentaire.

L'invention a également pour objet de fournir des composés alkylés dérivés des composés susmentionnés, conservant une capacité colorante et dont le caractère hydrosoluble est, le cas échéant, modifié par rapport à celui du composé colorant susmentionné.

L'invention a également pour but de fournir des composés susmentionnés pour la préparation de médicaments à activités antiradicalaire, antioxydante.

L'invention a pour objet les composés de formule I suivante :

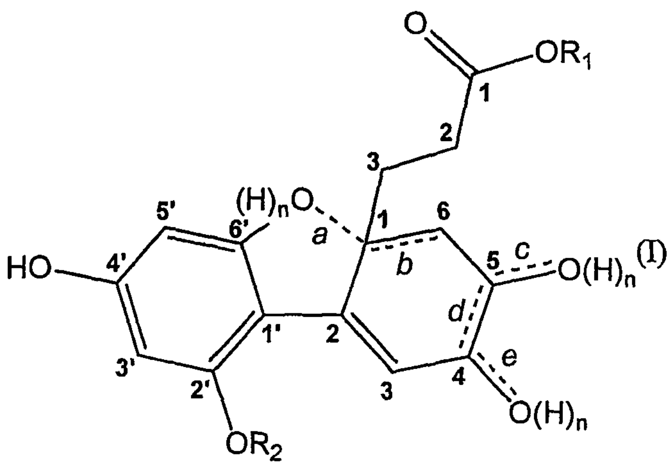

(I)

dans laquelle :

- n est égal à 0 ou 1,
- $R_1$ représente :

- un atome d'hydrogène,
- ou un groupe alkyle de 1 à 10 atomes de carbone,

- $R_2$ représente :

  - un atome d'hydrogène,
  - un groupe glucose, ou
  - un groupe xyloglucose,

et dans laquelle :

- lorsque n = 1, la liaison a n'existe pas, les liaisons **b** et **d** sont des doubles liaisons, et les liaisons c et e sont des liaisons simples, les composés correspondants étant désignés composés de formule I-**a**,
- lorsque n = 0, les liaisons **a, b** et **d** sont des liaisons simples, et les liaisons c et e sont des doubles liaisons, les composés correspondants étant désignés composés de formule I-**b**.

L'invention concerne plus particulièrement les composés définis ci-dessus, répondant à la formule I-**a** suivante :

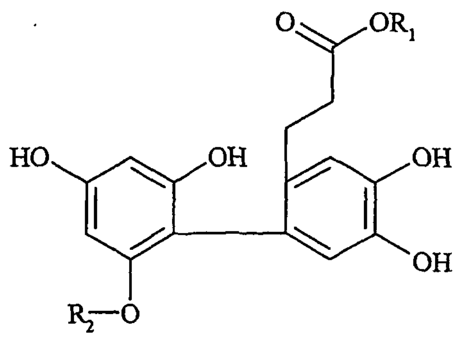

(I-a)

dans laquelle $R_1$ et $R_2$ sont tels que définis ci-dessus.

L'invention a plus particulièrement pour objet les composés définis ci-dessus, répondant à la formule I-**b** suivante :

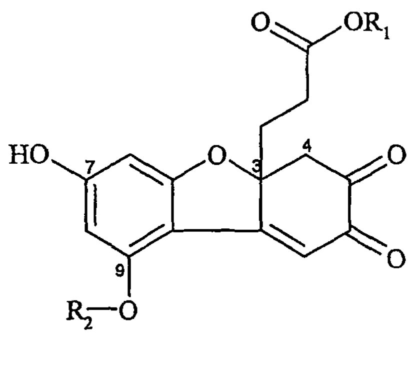

(I-b)

dans laquelle $R_1$ et $R_2$ sont tels que définis ci-dessus.

L'invention concerne plus particulièrement les composés de formule I-**a** ou I-**b** définis ci-dessus, dans laquelle $R_1$ représente H, un groupe méthyle $CH_3$, un groupe éthyle $CH_2$-$CH_3$, un groupe propyle $(CH_2)_2$-$CH_3$, un groupe isopropyle $CH(CH_3)$-$CH_3$, un groupe butyle $(CH_2)_3$-$CH_3$, un groupe hexyle $(CH_2)_5$-$CH_3$, ou un groupe octyle $(CH_2)_7$-$CH_3$, et $R_2$ est H, glucose, ou xyloglucose.

L'invention a plus particulièrement pour objet encore les composés de formule I-a-1 ou I-b-1 correspondant respectivement aux composés de formules I-a et I-b définis ci-dessus, dans lesquelles $R_1$ représente H, et $R_2$ représente

un glucose.

L'invention a également pour objet un procédé de préparation des composés susmentionnés, **caractérisé en ce qu**'il comprend une étape de mise en présence de phloridzine, pure ou sous forme d'extrait brut de végétaux, notamment de pomme ou de pommier (feuilles, rameaux, ou racines), avec une polyphénol oxydase (PPO) d'origine végétale, fongique ou bactérienne, à activité crésolase et catécholase en présence d'oxygène de l'air ou d'une atmosphère enrichie en oxygène, suivie le cas échéant d'une étape de purification du composé ainsi obtenu de formule I-**a** ou I-**b** dans laquelle $R_1$=H et $R_2$ est H, glucose, ou xyloglucose, et le cas échéant d'une étape de traitement de ce dernier composé avec un alcool de formule $R_1$-OH dans laquelle $R_1$ représente un groupe alkyle tel que défini ci-dessus, pour obtenir un composé de formule I-**a** ou I-**b** dans laquelle $R_1$ représente un groupe alkyle tel que défini ci-dessus, cette étape de traitement étant effectuée avant ou après l'étape de purification susmentionnée, et le cas échéant, une étape de purification du composé de formule I-**a** ou I-**b** dans laquelle $R_1$ représente un groupe alkyle tel que défini ci-dessus obtenu lors de l'étape précédente.

L'invention a plus particulièrement pour objet un procédé tel que défini ci-dessus,
**caractérisé en ce que** la PPO utilisée est un extrait enzymatique de végétaux comestibles, notamment de pomme, tel qu'obtenu par :

- broyage des tissus végétaux apprêtés ou non dans un milieu permettant d'éviter toute oxydation,
- éventuellement tamisage de la pulpe obtenue lors de l'étape précédente de manière à éliminer les particules de taille supérieures à 50 $\mu$m et obtenir une suspension homogène,
- micro-filtration tangentielle de la suspension susmentionnée afin de concentrer la PPO,
- lavage de la suspension concentrée en cours de microfitration tangentielle par addition de tampon additionné d'acide ascorbique jusqu'à élimination des substrats de la PPO,
- lavage de la suspension concentrée en cours de microfitration tangentielle par addition de tampon non additionné d'acide ascorbique jusqu'à élimination de l'acide ascorbique,
- récupération du rétentat de micro-filtration tangentielle, centrifugation, refroidissement et, le cas échéant, congélation dans l'azote liquide.

En variante, la PPO utilisée est un extrait enzymatique d'origine fongique ou bactérienne, telle que décrite dans Oszmianski et Lee, 1991.

La phloridzine utilisée dans le cadre du procédé susmentionné est sous forme pure (phloridzine commerciale), ce qui conduit à l'obtention de composés de formules Ia et/ou Ib dans lesquelles $R_1$=H et $R_2$ = glucose, ou sous la forme d'un extrait de pomme riche en phloridzine, par exemple l'extrait Pomactiv® HDH décrit ci-après, cet extrait étant susceptible de contenir également de la phlorétine, et/ou le xyloglucoside de phlorétine, conduisant respectivement à l'obtention de composés de formules Ia et/ou Ib dans lesquelles $R_1$=H et $R_2$ = H, et/ou $R_1$=H et $R_2$ = xyloglucose.

L'invention concerne également un procédé tel que décrit ci-dessus, **caractérisé en ce que** la purification des composés de formule I-**a** ou I-**b**, est effectuée par chromatographie liquide haute performance, avantageusement en phase inverse, ou par des procédés comprenant des étapes de filtration, centrifugation, et de chromatographie frontale basse pression.

L'invention a également pour objet toute composition contenant le composé de formule I-**a** ou I-**b** susmentionnée dans laquelle $R_1$=H, et $R_2$ est H, glucose, ou xyloglucose, telle qu'obtenue par mise en présence d'un extrait de pomme ayant une activité PPO tel qu'obtenu selon la méthode décrite ci-dessus, avec un extrait de phloridzine, de phlorétine, ou de xyloglucoside de phlorétine, de pomme ou de pommier, en présence d'oxygène de l'air ou d'une atmosphère enrichie en oxygène.

L'invention concerne plus particulièrement toute composition contenant un composé de formule I-**a** ou I-**b** susmentionnée dans laquelle $R_1$ représente un groupe alkyle tel que défini ci-dessus, et $R_2$ est H, glucose, ou xyloglucose, ladite composition étant telle qu'obtenue par traitement de la composition susmentionnée contenant le composé de formule I-**a** ou I-**b** dans laquelle $R_1$=H, avec un alcool de formule $R_1$-OH dans laquelle $R_1$ représente un groupe alkyle tel que défini ci-dessus.

L'invention a plus particulièrement pour objet toute composition telle que définie ci-dessus, **caractérisée en ce que** la proportion en poids des composés de formule I-**a** ou I-**b** dans ladite composition est d'environ 15% à environ 90%, notamment d'environ 50% à 80%.

L'invention concerne également toute composition alimentaire **caractérisée en ce qu'**elle comprend au moins un composé de formule I-**a** ou I-**b** susmentionnée, ou une composition telle que définie ci-dessus.

L'invention a également pour objet l'utilisation d'au moins un composé de formule I-**a** ou I-**b** susmentionnée, avantageusement de formule I-**b**, ou d'une composition telle que définie ci-dessus, en tant que colorant de couleur jaune à orange, notamment dans le cadre de l'alimentation humaine ou animale.

L'invention concerne également toute composition pharmaceutique ou nutraceutique **caractérisée en ce qu'**elle comprend au moins un composé de formule I-**a** ou I-**b** susmentionnée, avantageusement de formule I-a, ou une

composition telle que définie ci-dessus, le cas échéant en association avec un véhicule pharmaceutiquement acceptable.

L'invention a plus particulièrement pour objet toute composition pharmaceutique telle que définie ci-dessus, **caractérisée en ce qu**'elle se présente sous une forme administrable par voie orale, notamment sous forme de suspension buvable, ou de gélules ou comprimés.

L'invention concerne également l'utilisation d'au moins un composé de formule I-**a** ou I-**b** susmentionnée, avantageusement de formule I-a, ou d'une composition telle que définie ci-dessus, pour la préparation d'un médicament à activité antioxydante et antiradicalaire, destiné notamment à la prévention ou au traitement du stress oxydatif et des pathologies liées au stress oxydatif, intervenant notamment dans la diminution du risque d'apparition de maladies cardiovasculaires et de cancers.

L'invention concerne également toute composition cosmétique **caractérisée en ce qu**'elle comprend au moins un composé de formule I-**a** ou I-**b** susmentionnée, avantageusement de formule I-a, ou une composition telle que définie ci-dessus.

Avantageusement, les compositions cosmétiques susmentionnées se présentent sous forme de crèmes, ou de gels.

L'invention a également pour objet toute méthode de traitement esthétique, **caractérisé en ce qu'**elle comprend l'application sur la peau d'un individu d'une composition cosmétique susmentionnée.

L'invention sera davantage illustrée à l'aide de la description détaillée qui suit de la préparation de nouveaux colorants jaunes orangés issus de l'oxydation enzymatique de la phloridzine, à savoir de l'acide 3-(4'-hydroxy-2'-glucosyl-4,5-dioxo-3,6-dihydro-2H-dibenzofuran-2-yl)-propionique ou POP de formule I-**b-1** dans laquelle $R_1$ = H, et $R_2$ = glucose et de ses dérivés de formule I-**b** dans laquelle $R_1$ représente un groupe alkyle tel que défini ci-dessus. L'invention sera aussi illustrée par la mise en évidence et la caractérisation structurale des composés de formule I-**a-1** à savoir l'acide 3-(4,5,4',6'-tetrahydroxy-2'-glucosyl-biphenyl-2-yl)-propionique qui est un précurseur du colorant de formule I-**b-1**. Le terme POP sera utilisé pour décrire la préparation colorante contenant les composés de formule I-b-1 et I-a-1, avantageusement de formule I-**b-1.**

Comme cela avait déjà été observé par Oszmianski et Lee (1991), l'oxydation enzymatique de la phloridzine présente un temps de latence avant l'apparition de la couleur correspondant au produit jaune de formule I-**b-1**. L'oxydation enzymatique de la phloridzine semble se faire de façon quasiment séquentielle. L'oxydation de la phloridzine forme d'abord la 3-hydroxy-phloridzine (étape limitante) qui est rapidement consommée pour former le composé I-**a-1**. Celui-ci est ensuite réoxydé une nouvelle fois pour former le produit I-**b-1** plus lentement. Il semble que le produit I-**b-1** ait donc une affinité plus faible pour l'activité catécholase de l'extrait PPO que la 3-hydroxyphloridzine.

A partir de ce constat plusieurs solutions techniques permettent de produire une préparation plus ou moins riche en composés colorants de formule I**-b- 1**, ou en son précurseur direct de formule I-**a-1 :**

- limiter le temps de réaction à environ 2h dans les conditions décrites ci-après permet d'accumuler les composés I-**a-1** avant qu'ils soient de nouveau oxydés en I-**b-1.**
- l'ajout d'acide ascorbique dans le milieu réactionnel permet d'accumuler à la fois de la 3-hydroxy-phloridzine, et les composés I-**a**, en ralentissant la dernière étape d'oxydation par son pouvoir réducteur.

Le colorant POP (mélange contenant avantageusement le composés de formule I-**b-1** dans laquelle $R_1$=H, et $R_2$=glucose) présente un intérêt en tant que colorant jaune naturel. Il peut être obtenu dans un milieu réactionnel biotechnologique, en contrôlant l'oxydation enzymatique de la phloridzine catalysée par l'activité polyphénoloxydase (PPO), telle qu'elle se déroule naturellement dans la production d'un jus de pomme :

<u>Préparation de l'extrait enzymatique de PPO:</u>

Le parenchyme végétal, par exemple la pulpe de pomme, est broyé dans un milieu permettant d'éviter toute oxydation (pH acide, froid, acide ascorbique). Les particules solides de taille supérieure à 50 μm sont alors éliminées par tamisage afin d'obtenir une suspension homogène. Celle-ci est ensuite soumise à une micro-filtration tangentielle sur membrane (0,45 μm) avec apport d'une solution tampon additionnée d'acide ascorbique pour éliminer les substrats polyphénoliques de la PPO. Enfin, le rétentât récupéré est lavé à l'aide d'un tampon sans acide ascorbique, puis centrifugé. Le culot de centrifugation est dispersé dans un minimum de tampon. Cette préparation enzymatique de PPO doit alors être conservée au froid en vue d'une utilisation rapide ou être congelée dans l'azote liquide puis conservée à -25°C pour une utilisation ultérieure.

<u>Mécanisme de formation</u> : Un extrait de phloridzine mis en présence de cet extrait de PPO. L'activité crésolase, permet la formation de 3-hydroxyphloridzine. Ce dernier composé est oxydé (par l'activité catécholase de la PPO) puis réagit immédiatement de façon intramoléculaire puis avec l'eau pour former une nouvelle structure (composé de formule I-**a-1** dans laquelle $R_1$=H, et $R_2$ = glucose). Cette dernière molécule est à nouveau oxydée par la PPO pour conduire au composé jaune final (composé de formule I-**b-1** dans laquelle $R_1$=H, et $R_2$ =glucose).

Spécificités de structure : La formule chimique présente une forte originalité structurale par la présence d'une fonction acide carboxylique qui confère à la molécule un caractère fortement hydrophile. Cette fonction est aisément estérifiable par divers groupements chimiques pour donner de nouvelles structures plus ou moins hydrosolubles, ou pouvant apporter de nouvelles fonctionnalités, sans affecter les propriétés colorantes.

Contrairement au principal colorant jaune hydrosoluble alimentaire actuellement utilisé - la tartrazine (E102) - qui est issue de synthèse chimique, le colorant en question est dérivé de la phloridzine par un procédé enzymatique et correspond à une partie de la coloration naturelle d'un jus de pomme.

Il existe d'autres colorants jaunes naturels tels que la curcumine ou la quercétine, cependant leur solubilité limite leur utilisation en milieu aqueux (logP positif, avec P coeff. de partage [*n*-octanol/eau]), rendant nécessaire leur inclusion dans des complexes hydrosolubles (β-cyclodextrines, EP0612814). Au contraire le colorant POP est librement soluble dans l'eau (logP négatif). De plus, cette mauvaise solubilité des colorants actuels les rend « tachants »(WO 01/91585A2) alors que le colorant POP est facilement lavable à l'eau.

D'une part le colorant POP présente une grande capacité de coloration comme en témoigne le coefficient d'extinction molaire de la molécule de formule I-b-1, ε=17 400 $DO.mol^{-1}.L^{-2}.cm^{-1}$ à pH 3, et cette capacité augmente avec le pH. Une solution à 20 ppm de ce composéest jaune intense.

D'autre part, des mesures colorimétriques avec un spectrophotomètre Minolta montre que le composé de formule I**-b- 1**, composant majeur du colorant POP :

- apporte une couleur jaune stable (teinte CIE = 10) pour un pH inférieur à 5-6, ce qui correspond à la plupart des produits agro-alimentaires, et orange (teinte CIE = -50) pour les pH supérieurs,
- permet de conserver une grande luminosité au produit à colorer, la luminosité de l'eau distillée fixée à 100 ne diminue pratiquement pas, pour des concentrations allant jusqu'à 100 ppm,
- permet d'obtenir des couleurs vertes (type sirop de menthe verte) en mélange avec des colorants bleus, de la même façon que la tartrazine.

**Obtention, structure et quelques propriétés du colorant POP**

**A. Principes généraux de l'obtention du pigment POP :**

Le pigment jaune, dénommé POP (i.e. Produit d'Oxydation de la Phloridzine ) est produit par oxydation enzymatique de la phloridzine, composé phénolique naturellement présent dans la pomme et spécifique de ce fruit. L'enzyme impliquée est la polyphénoloxydase, également présente naturellement dans la pomme mais dans un compartiment cellulaire (plastes) différent de celui de la phloridzine et des autres substrats phénoliques (vacuoles).

**Pour l'obtention d'une composition contenant une concentration significative en POP, il est avantageux :**

- d'utiliser comme substrat de départ la phloridzine à l'état purifié ou tout au moins une préparation hautement concentrée en phloridzine. Les préparations utilisées dans le cadre de la présente invention contiennent de préférence plus de 40% de phloridzine.
  Une préparation particulièrement avantageuse utilisée dans le cadre de la présente invention, dénommée ci-après Pomactiv® HDH, contient 51 % de polyphénols dont :

  * 96% de dihydrochalcones, à savoir 73% de phloridzine, 2% de xyloglucoside de phlorétine, et 21 % de phlorétine,
  * 1% d'acides hydroxycinnamiques,
  * 3% de flavonols.

- d'utiliser une préparation enzymatique de type polyphénoloxydase (PPO) riche en activité crésolase. En fait, la polyphénoloxydase se décompose en une activité crésolase (permettant l'hydroxylation de la phloridzine) et une activité catécholoxydase (permettant l'oxydation de la phloridzine hydroxylée). Un procédé d'obtention d'une préparation de PPO particulièrement avantageuse est décrit ci-après.

L'obtention proprement dite de la préparation colorante POP peut être réalisée de deux façons :

1. à partir de phloridzine commerciale purifiée : dans ce cas, le composé de formule I**-b-1** peut constituer plus de 50 % de la préparation obtenue, les autres constituants correspondant à un pool d'autres produits d'oxydation dont

le composé de formule I-**a-1** n'ayant pas ou peu de capacité colorante,

2. à partir de Pomactiv HDH mis en incubation avec la préparation PPO en solution et avec agitation à l'air. La procédure permet d'obtenir une oxydation quasi totale de la phloridzine avec une conversion en pigment POP supérieure à 50 %. Les autres constituants de cette préparation correspondent pour une part aux composés autres que la phloridzine initialement présents dans Pomactiv HDH et d'autre part aux intermédiaires réactionnels semblables aux précédents.

La purification de la molécule colorante de formule I-**b- 1** dans laquelle $R_1$=H, et $R_2$=glucose, peut être réalisée par chromatographie liquide haute performance en phase inverse. Le détail du protocole est exposé ci-après.

## B. Production d'un extrait enzymatique avec une activité crésolase

A titre d'exemple la méthode d'extraction de PPO à partir de pulpe végétale a été appliquée à la pulpe de pomme comme indiquée ci-dessous :

L'activité crésolase de la PPO de pomme a été extraite de 4 kg de pomme *Idared*, placés dans 20 L d'eau additionnée d'acide ascorbique à 20 mM, soit 3,5 $g.L^{-1}$ (cf schéma 1 suivant). La compote obtenue par broyage fin, a ensuite été tamisée, micro-filtrée, rinçée puis centrifugée et reprise dans 50 mL d'eau. Ainsi l'activité crésolase de l'enzyme peut-être récupérée à partir de pommes (commerciales issues de chambre froide), sans précaution particulière au niveau de l'ajustement du pH.

*Schéma 1.*

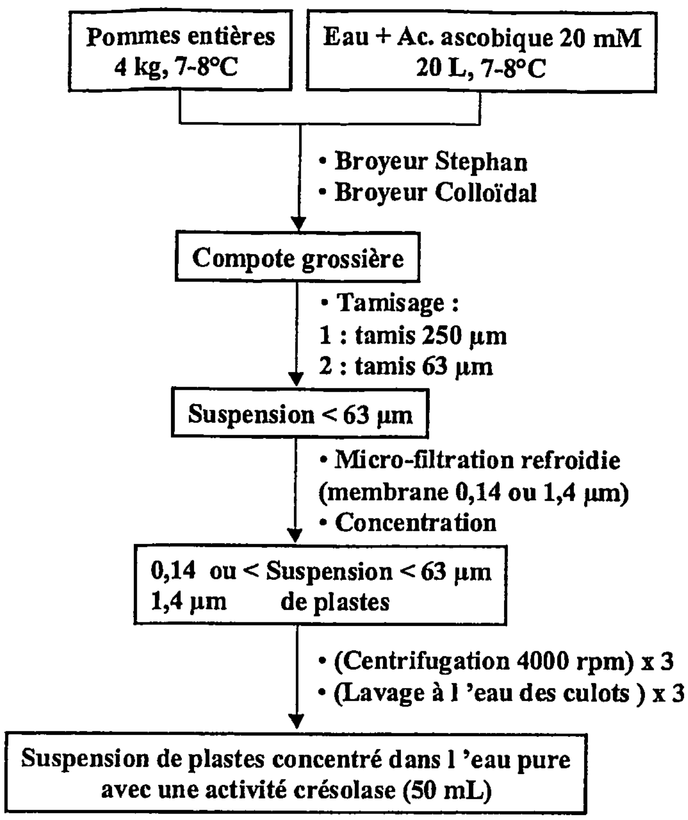


## C. Conduite de l'oxydation

L'extrait Pomactiv® HDH contenant une forte proportion de phloridzine a été utilisé comme substrat de l'oxydation, celui-ci se disperse plus facilement dans l'eau que de la phloridzine commerciale pure utilisée jusqu'à présent.

L'oxydation a été conduite dans un volume de 1,37 L d'eau contenant env. 1 mM de phloridzine, placé dans un fermenteur de microbiologie avec un bullage d'air comprimé et une agitation avec une double hélice marine à 250

rpm. L'ensemble ainsi réglé permet au système de rester saturé en air. L'oxydation a été menée pendant 15 h à 30°C. La solution a été clarifiée par centrifugation puis lyophilisée.

Bilan de l'oxydation :

- Récupération de matière : 90 %.
- Conversion de la phloridzine en POP : 50 % (en masse) ; 47 % en moles

A ce stade, le lyophilisat correspond à une préparation colorante contenant 26 % de la molécule de formule **I-b-1.**

**D. Purification du composé colorant de formule I-b-1 par Chromatographie liquide à l'échelle semi-préparative.**

Echantillon : 200 mg du lyophilisat issu de l'oxydation (cf § C ci-dessus) sont solubilisés dans l'eau acidifiée (2,5 % acide acétique) à la concentration de 20 $g.L^{-1}$.

Système chromatographique haute performance :

- système de pompage à gradient constitué de 2 pompes Dynamax SD 200 avec débit maximum de 100 $mL.min^{-1}$ ; solvant A : acide acétique dilué (2.5 % v/v) ; solvant B : acétonitrile pur.
- débit : 35 $mL.min^{-1}$ ,
- une colonne Novapack C18, 6 μm de dimension 25 x 100 mm avec une précolonne de 25 x 20 mm ; - gradient : initial : 3 % B ; puis 3 min : 3% B ; puis 5 min : 8% B ; puis 10 min : 15 % B, puis 12 min : 90 % B ; puis 14 min : 90 % B ; et enfin 16 min : 3% B,
- un détecteur UV-visible réglé sur 400 nm (détection dans le jaune).

Injections : 3 injections de 3 ml de la solution ont été effectuées (soit 3 x 60 mg de lyophilisat).

Collecte : le pigment est détecté en sortie de colonne entre 400 et 550 secondes d'élution ; la fraction d'éluât correspondante a été collectée pour chacune des 3 injections. Les volumes des 3 collectes ont été rassemblés.

Évaporation et lyophilisation : la fraction collectée a été traitée à l'évaporateur en pression réduite afin d'éliminer le solvant organique (i.e. acétonitrile). La fraction aqueuse résiduelle a été congelée, lyophilisée puis pesée.

**Bilan :** 51 mg de composé de formule I-**b- 1** (POP purifié) ont été obtenus soit un rendement de purification de 28,3 %.

**E. Analyse structurale du composé de POP purifié et de son intermédiaire incolore**

L'analyse structurale a été réalisée sur la molécule colorante et sur son intermédiaire incolore purifiés. Elle a permis de démontrer que ces composés correspondait à des structures originales (I-**b-1** et I-**a-1** dans lesquelles $R_1$=H, et $R_2$=glucose), notamment par la présence d'une fonction acide carboxylique initialement absente de la phloridzine.

**F. Dérivatisation (estérification) par divers alcools.**

Mode opératoire :

- pesée précise de 1 à 2 mg de la fraction POP non purifiée (26 % de molécule de formule I-**b-1**) telle que décrite au § C sont incubés avec l'alcool acidifié par HCl (0,5N final) à 50 °C pendant 1 h. Le volume d'alcool est ajusté de façon à avoir une concentration finale en fraction POP de 1 $g.L^{-1}$. Les alcools suivants ont été testés : méthanol, propanol-1, propanol-2, butanol-1, butanol-3, hexanol-1, octanol-1.

Confirmation de la conversion en esters : la formation des esters correspondant a été montrée par HPLC couplée à la spectrométrie de masse en mode négatif et avec également en série une détection à 420 nm (jaune). Les rendements de conversion ont été calculés sur la base des chromatogrammes à 420 nm en effectuant le rapport des aires du pic de l'ester sur celle du pic du composé de formule **I-b- 1** dans les mêmes conditions de concentration (voir

Tableau I).

**Tableau I: Temps de rétention chromatographique, ions pseudo-moléculaires et rendements de conversion du composé de formule I-b-1 et de ses dérivés estérifiés.**

| Composé | Temps de Rétention* (min) | Ion $[M-H]^-$ | Rendement** de conversion en ester (%) | DO 420 nm (jaune) du milieu réactionnel dilué à 0,1 g/l |
|---|---|---|---|---|
| I-b-1 | 13,5 | 465 | - | 0,973 |
| Ester méthylique de I-b-1- | 17,9 | 479 | 100 | 0,922 |
| Ester propylique | 23,9 | 507 | 95 | 0,945 |
| Ester isopropylique | 23,9 | 507 | 32 | 0,890 |
| Ester butylique | 26,8 | 521 | 100 | 0,978 |
| Ester hexylique | 32,0 | 549 | 44 | 0,615 |
| Ester octylique*** | 37.1 | 577 | 16 | 0,230 |
| * en HPLC en phase inverse avec gradient d'acétonitrile ; ** estimé sur la base de l'aire du pic chromatographique de l'ester à 400 nm par rapport à l'aire du pic du composé de formule I-b-1 à 400 nm dans les mêmes conditions ; *** La fraction POP n'était que partiellement soluble dans cet alcool, en conséquence, les valeurs de rendements et de DO sont vraisemblablement affectées par ce phénomène. | | | | |

Conclusion: on remarque que les temps de rétention sont d'autant plus longs que l'alcool utilisé est « lourd » traduisant de ce fait la formation d'un pigment d'autant plus apolaire. L'estérification du composé colorant par une série d'alcools aliphatiques se fait avec des rendements qui varient de 16 à 100 % en conservant généralement la capacité colorante (voir DO 420 nm). Il est donc aisément possible de convertir le composé colorant en d'autres pigments moins hydrosolubles pour d'autres applications (cosmétiques...).

**G. Capacité antioxydante *in vitro* des composés de formule I-a-1 et I-b-1**

La capacité antioxydante de la molécule de formule I-**a-1** (précurseur incolore) et I-**b-1** (pigment jaune) a été déterminée sur ces molécules purifiées selon un protocole similaire à celui décrit au paragraphe D. La pureté des composés mesurée par CLHP, était supérieure à 98 %. Deux méthodes de mesure de la capacité antioxydante *in vitro* ont été utilisées : la méthode DPPH, d'après Brand-Williams *et al.* (1995) et la méthode FRAP (Ferric Reducing Antioxidant Power) d'après Pulido *et al.* (2000). Pour comparaison ou pour l'expression des résultats, les capacités antioxydantes des composés suivants ont été également mesurées : vitamine C (acide ascorbique), Trolox (analogue hydrosoluble de la vitamine E), (-)-épicatéchine, et phloridzine.

Les résultats sont présentés dans les tableaux II et III ci dessous.

**TABLEAU II - MESURE DE LA CAPACITE ANTIOXYDANTE EN MILIEU METHANOLIQUE PAR LA METHODE DPPH (D'APRES BRAND-WILLIAMS *ET AL.*, 1995)**

| | $EC_{50}$* | ARP* | TEAC *** |
|---|---|---|---|
| **Trolox** | 0,203 | 4,93 | 1,00 |
| **Vitamine C** | 0,206 | 4,85 | 0,98 |
| **(-)-épicatéchine** | 0,181 | 5,52 | 1,12 |
| **Phloridzine** | 2,33 | 0,43 | 0,09 |
| **I-a-1** | **0,258** | **3,87** | **0,78** |

(suite)

| | $EC_{50}$* | ARP* | TEAC *** |
|---|---|---|---|
| **I-b-1** | **2,27** | **0,44** | **0,09** |

* $EC_{50}$ = Rapport de concentrations entre l'antioxydant (mol/L) et le DPPH (mol/L) qui permet le piégeage de 50 % des radicaux DPPH initialement présents. ARP (antiradical power) = $1/EC_{50}$.
*** TEAC (Trolox équivalent antioxidant capacity) = capacité antioxydante exprimée par rapport à celle Trolox.

**Tableau III - Mesure de la capacité antioxydante en milieu aqueux par la méthode FRAP pour 10 minutes d'incubation (d'après Pulido *et al.*, 2000).**

| | $EC_1$* (en μmol/L) | TEAC* |
|---|---|---|
| **Trolox** | 411 (10) | 1.00 (0.02) |
| **Vitamine C** | 400 (9) | 1.03 (0.02) |
| **(-)-épicatéchine** | 359 (11) | 1.14 (0.03) |
| **Phloridzine** | 5 161 (195) | 0.08 (0.00) |
| **I-a-1** | **477 (15)** | **0,86 (0.03)** |
| **I-b-1** | **7 754 (235)** | **0,05 (0.00)** |

* EC = concentration en antioxydant (en μmol/L) ayant un pouvoir réducteur équivalent à 1 mmol/L de $FeSO_4$ 7 $H_2O$ (les valeurs correspondent à la moyenne de 3 déterminations. TEAC = capacité antioxydante exprimée en équivalant Trolox. Les données entre parenthèses correspondent à l'écart-type.

**L'expression des valeurs de la capacité antioxydante en équivalent Trolox (TEAC) montre une très bonne cohérence des résultats entre les deux méthodes.**

Les valeurs de capacité antioxydante obtenues pour les principaux standards sont également cohérentes avec celles décrites dans la littérature. Ainsi, par la méthode FRAP, la (-)-épicatéchine, la vitamine C, et le Trolox ont donné respectivement des valeurs EC1 de 359, 400 et 411 qui sont à comparer avec les valeurs respectives de 348, 392 et 322 obtenues par Pulido *et al.* (2000). Les valeurs de EC 50 obtenues par la méthode au DPPH (soit 0,181, 0,206 et 0,203 respectivement pour la (-)-épicatéchine, la vitamine C, et le Trolox) s'écartent davantage mais restent cependant du même ordre que celles obtenues par Lu et Foo en 2000 soit 0,135, 0,35 et 0,30 pour ces composés, respectivement.

La molécule de formule I-**b-1** (pigment jaune) n'a présenté qu'une faible capacité antioxydante. Celle-ci est du même ordre que celle de la phloridzine elle même très faible comparée à celle d'autres polyphénols de la pomme tels que la (-)-épicatéchine.

En revanche, la molécule de formule 1-**a-1** (précurseur incolore) a montré une capacité antioxydante importante (10 fois supérieure à celle de son précurseur la phloridzine) qui demeure toutefois légèrement plus faible que celle d'autres standards tels que la (-)-épicatéchine, la vitamine C ou le Trolox.

## H. Paramètres de couleur et stabilité de la couleur en fonction du pH

Le coefficient d'extinction molaire du colorant purifié (composé de formule **I-b- 1)** a été estimé à 17400 $mol^{-1}.cm^{-1}$ (420 nm à pH 3). Le spectre UV-visible d'une solution de préparation POP a été mesuré pour différents pH entre 2 et 8 (voir figure 1). Des mesures de couleur (chromamètre Minolta) ont également été réalisées dans cette zone de pH. Les résultats détaillés des paramètres colorimétriques sont consignés ci-après.

### Paramètres Colorimétriques du colorant POP purifié (composé de formule I-b-1)

Les paramètres de coloration du colorant par rapport à de l'eau distillée (référence) ont été mesurés en solution dans un tampon de Mc Ilvaine (citrate-phosphate) à différents pH (2,2 à 8) et ce, pour plusieurs concentrations.

Les mesures ont été réalisées à l'aide d'un spectrophotomètre Minolta CM-3600d (©1998 Minolta Co., Ltd. Japon) avec la plaque de calibration (Minolta n° 13171008). Le spectrophotomètre équipé d'un logiciel de retraitement SpectraMagic V 3.60G (Cyberchrome Inc Minolta Co. Ltd), a été paramétré pour utiliser l'espace colorimétrique CIE L*a*b (Figure 2), avec l'illuminant D65-10° (D65-A-F2), et l'observateur moyen normalisé 10° CIE 1964 (sensibilité d'un oeil moyen). A partir des valeurs a* et b* nous avons calculé les coordonnées polaires

$$C^* = \sqrt{(a^*)^2 + (b^*)^2} \quad \text{et} \quad h = \tan g^{-1}\left(\frac{a^*}{b^*}\right) \times \frac{180}{Pi}$$

Dans la suite du texte nous exploiterons principalement ces coordonnées polaires C* et h, car elles ont chacune une signification colorimétrique : C* est la distance au centre de l'espace et correspond à la saturation de la couleur alors que h est un angle (exprimé ici en degré) qui définit la teinte. A titre indicatif pour la couleur rouge la teinte se situe au voisinage de 0°, pour le jaune environ 90°, pour le vert 180° et pour le bleu 270°. La valeur L* est utilisée telle quelle et exprime la clarté.

**Effet du pH sur la coloration**

**Tableau IV : Paramètres colorimétriques de POP purifié (composé de formule I-b-1 à env. 20 mg/L) dans un tampon de Mc Ilvaine en fonction du pH (L* : luminosité ; a* : axe vert-rouge ; b* : axe bleu-jaune ; C* : saturation)**

| Coordonnées L*a*b* | L* | a* | b* | C* | h | Teinte CIE |
|---|---|---|---|---|---|---|
| Référence : H2Od | 100,0 | 0,0 | 0,0 | 0,0 | - | 0,0 |
| POP à pH 2,2 | 98,7 | -13,5 | 40,7 | 42,9 | 108,4 | 11,2 |
| POP à pH 3 | 98,5 | -13,4 | 41,1 | 43,2 | 108,1 | 11,0 |
| POPàpH4 | 98,4 | -13,2 | 42,3 | 44,3 | 107,3 | 10,2 |
| POP à pH 5 | 97,5 | -11,4 | 46,7 | 48,1 | 103,8 | 5,9 |
| POP à pH 6 | 93,5 | -0,1 | 57,7 | 57,7 | 90,1 | -20,5 |
| POP à pH 7 | 88,1 | 16,3 | 71,4 | 73,2 | 77,2 | -63,5 |
| POP à pH 8 | 87,1 | 19,5 | 72,2 | 74,8 | 74,9 | -71,8 |

Les coordonnées du colorant mesurées par rapport à l'eau distillée sont regroupées dans le tableau IV. Pour les pH acides (<5) le colorant POP diminue peu la luminosité par rapport à la luminosité de référence de l'eau distillée et agit quasi-uniquement sur la teinte et la saturation.

Les valeurs de h comprises entre 75 et 110 indiquent que la teinte jaune (*) est largement majoritaire avec des nuances légèrement vertes pour les pH les plus acides, et tend vers l'orange pour les pH supérieurs à 6. Il faut remarquer cependant une grande constance de la teinte entre pH 2.2 et pH 5 c'est à dire dans la plage de pH ou se situent la majorité des produits alimentaires. Une augmentation du pH a également pour effet d'accentuer la saturation de la couleur simultanément à la modification de teinte.

L'effet du pH sur le spectre UV-visible du colorant en absorbance, est représenté sur la figure 1.

L'effet du pH sur le spectre visible du colorant en % de transmittance, est représenté sur la figure 3.

**Effet de la concentration sur la coloration**

Une étude colorimétrique comparable a été effectuée à pH 3 pour des concentrations variables (Tableau V).

**Tableau V : Paramètres colorimétriques de POP purifié (composé de formule I-b-1) dans un tampon de Mc Ilvaine à pH 3 en fonction de la concentration (L* : luminosité ; a* : axe vert-rouge; b* : axe bleu-jaune ; C* : saturation)**

| POP à pH 3 | L* | a* | b* | C* | h | Teinte CIE |
|---|---|---|---|---|---|---|
| H2Od | 100,0 | 0,0 | 0,0 | 0,0 | - | 0,0 |
| POP 5 mg/L | 99,6 | -4,6 | 11,5 | 12,4 | 111,7 | 3,5 |
| POP 10 mg/L | 99,3 | -8,5 | 22,8 | 24,3 | 110,4 | 6,7 |
| POP 20 mg/L | 98,7 | -13,6 | 41,5 | 43,7 | 108,1 | 11,1 |

(suite)

| POP à pH 3 | L* | a* | b* | C* | h | Teinte CIE |
|---|---|---|---|---|---|---|
| H2Od | 100,0 | 0,0 | 0,0 | 0,0 | - | 0,0 |
| POP 40 mg/L | 97,9 | -17,7 | 67,1 | 69,4 | 104,8 | 14,3 |
| POP 100 mg/L | 96,5 | -17,8 | 96,0 | 97,6 | 100,5 | 10,4 |

De la même façon que lors de la variation de pH, la luminosité des solutions du colorant ne diminue que très faiblement en fonction de la concentration. La teinte reste dans les jaunes quelle que soit la concentration avec une légère diminution des nuances vertes lorsque la concentration augmente.

La saturation de la couleur est bien corrélée avec la concentration de colorant jusqu'à la concentration de 20 $mg.L^{-1}$ (Figure 4), Au-delà de 40 $mg.L^{-1}$, le détecteur sature, l'absorbance de la solution atteignant valeur de 3 à 400 nm (Figure 5). Cette corrélation permet d'affirmer qu'une augmentation de 1 $mg.L^{-1}$ de colorant en solution à pH 3 équivaut à un gain d'environ 2 points de saturation.

Conclusion : Le spectre UV-visible est relativement stable pour des pH compris entre 3 et 5 (ce qui correspond à la majorité des aliments). Pour des pH supérieurs, la couleur devient plus orangée tandis qu'elle devient jaune pâle avec une légère nuance verte pour un pH inférieur à 3. En conclusion, la couleur du colorant en solution est au moins aussi stable sinon supérieure à celle de la tartrazine.

**I. Solubilité dans l'eau**

La remise en solution de la fraction POP non purifiée (telle que décrit au § C.) s'avère soluble librement dans l'eau à des concentrations pouvant aller jusqu'à 100 $g.L^{-1}$. Pour des concentrations aussi élevées la couleur obtenue est noire, avec des reflets oranges. Cette forte capacité à la solubilisation en solution aqueuse confirme l'estimation par le calcul des coefficients de partage (octanol/eau) des composés de formule I-**b-1** et I-**a-1**. Ceux-ci sont en effet négatifs, indiquant la forte hydrosolubilité du colorant POP.

**J. Précurseur de pigmentation cutanée**

Shoji et de ses collaborateurs en 1997 ont montré que la phloridzine est lentement oxydée par la tyrosinase de la peau pour former un pigment jaune-orange probablement de structure similaire aux composés de formule I-**b**, constituant un protecteur contre les radiations UV. Leur formation en cosmétologie au niveau cutané permet donc d'utiliser la phloridzine comme un substrat autobronzant.

Comme nous avons pu le constater expérimentalement, l'étape limitante de la chaîne réactionnelle d'oxydation reste l'hydroxylation de la phloridzine par l'activité crésolase. Une préparation contenant les composés de formule I-**a-1** constitue un substrat direct de l'activité catécholase de la tyrosinase de la peau, et permet donc le développement d'une pigmentation beaucoup plus rapidement qu'à partir de la phloridzine de départ.

Au cours des réactions présentées précédemment il est possible de favoriser la formation du composé de formule I-**a-1** en arrêtant l'oxydation par filtration (élimination de l'extrait insoluble de PPO après 2h), ou en apportant un pouvoir réducteur au milieu réactionnel par ajout d'acide ascorbique (5 à 20 mM). La structure de base des composés de formule I-**a-1** peut en outre, être estérifiée par des alcools lourds ($R_1$), déglycosylée ($R_2$) par une hydrolyse acide ménagée, la rendant ainsi plus apolaire, ce qui la rend plus apte à pénétrer la barrière cutanée. Dans le cas d'une formulation cosmétique comprenant uniquement des extraits naturels tels qu'il peut s'en former dans un jus de pomme, la structure de base de formule I-**a-1** avec $R_1$ = H et $R_2$ = glucose, doit être formulée dans une émulsion grasse constituée de liposomes.

**K. Test de cytotoxicité sur modèle cellulaire**

SYSTEME D'ESSAI

Cellules TC-7 (sous clone de caco-2) cultivées sur nacelles (membrane en polyéthylène téréphtalate), utilisées à 21 jours de confluence, fournie par Bioprédic International

Emploi des cellules au passage 21.

PREPARATION DES PRODUITS A L'ESSAI

Produit "POP" purifié (composé de formule I-**b-1**), obtenu tel que décrit précédemment (paragraphe D) : solubilisé directement dans le tampon apical, testé à 20, 100, 200, 1000 et 2000 µM pour le test de cytotoxicité.
Pas de solvant utilisé pour la solubilisation des produits à l'essai.

PROTOCOLE D'INCUBATION

Système réactionnel : cultures de cellules TC-7 incubées en présence des produits à l'essai aux différentes concentrations citées ci-dessus.
Temps d'incubation : 2 heures.

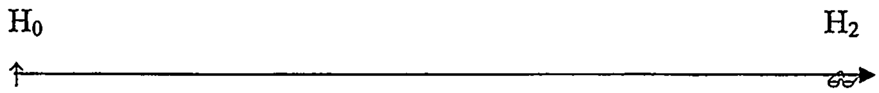


↑ : incubation des produits à l'essai
: évaluation des effets
Replicate : 5 par concentrations.
Température d'incubation : 37°C.
Autres conditions : atmosphère humide contenant 5 % de CO2.
Témoin : correspondant aux cellules incubées en absence de produit à l'essai

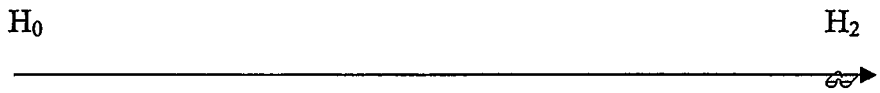


Replicate : 10

EVALUATION DES EFFETS

Viabilité cellulaire mesurée selon la méthode du rouge neutre après incubation avec les produits à l'essai :

- incubation en présence de rouge neutre pendant 3 heures
- lyse des cellules et lecture de la densité optique avec un spectrophotomètre à 540 nm (la densité optique du rouge neutre est proportionnelle au nombre de cellules vivantes).

TRAITEMENT DES DONNEES

Expression des résultats : en unités arbitraires de D.O.(densité optique) par échantillon et en pourcentage de variation par rapport au groupe témoin.

RESULTATS (Tableau VI)

**TABLEAU VI : EFFET DE POP PURIFIE SUR LA VIABILITE CELLULAIRE DES 5CELLULES TC-7**

| | Témoin | | Produit POP purifié (μM) | | | | |
|---|---|---|---|---|---|---|---|
| | | | 20 | 100 | 200 | 1000 | 2000 |
| DO | 0,456 | 0,485 | 0,441 | 0,432 | 0,407 | 0,438 | 0,442 |
| | 0,479 | 0,473 | 0,477 | 0,519 | 0,460 | 0,521 | 0,436 |
| | 0,483 | 0,476 | 0,501 | 0,517 | 0,480 | 0,518 | 0,458 |
| | 0,480 | 0,524 | 0,517 | 0,533 | 0,495 | 0,535 | 0,476 |
| | 0,469 | 0,519 | 0,510 | 0,518 | 0,495 | 0,509 | 0,429 |
| **Moyenne** | **0,484** | | **0,489** | **0,504** | **0,467** | **0,504** | **0,448** |
| Ecart type | 0,022 | | 0,031 | 0,041 | 0,037 | 0,038 | 0,019 |
| **% Témoin** | **100** | | **101** | **104** | **96** | **104** | **93** |

Aux concentrations testées , le produit POP purifié (composé de formule I-**b-1**) n'a pas eu d'effet sur la viabilité cellulaire des cellules TC-7. Aucun effet inhibiteur de référence n'a été noté jusqu'à 200 μM. A une concentration de 1000 μM, une légère diminution de la viabilité cellulaire a été mise en évidence.

**Légende des figures**

Figure 1 : Effet du pH sur le spectre UV-visible du colorant POP purifié (longueur d'onde en nm en abscisse) en absorbance (en ordonnée).

Figure 2 : Espace colorimétrique CIE L*a*b*

Figure 3 : Effet du pH sur le spectre visible du colorant POP purifié (longueur d'onde en nm en abscisse) en % de transmittance (en ordonnée).

Figure 4 : Corrélation de la saturation (en ordonnée) avec la concentration de colorant POP purifié (en abscisse).

Figure 5 : Effet de la concentration en colorant POP purifié sur l'absorbance (en ordonnée) dans le visible (longueur d'onde en nm en abscisse).

**Bibliographie**

Brand-Williams W., Cuvelier M.E., Berser C. Use of a Free Radical Method to Evaluate Antioxidant Activity, Lebensin.-Wiss. u.-Tecfinol., 1995, 28, 25-30.

Frerichs H., Ball E.G. (1964). Studies on the metabolism of Adipose Tissue. XVI. Inhibition by phlorizin and phloretin of the insulin-stimulated uptake of glucose. Biochemistry. 3, 7, 981-985

Lea, A. G. H. (1984). Farb- und gerbstoffe in englischen mostapfeln. Flüssiges Obst, 8, 356-361.

Lu Y.and Foo L. Y. Antioxidant and radical scavenging activities of polyphenols from apple pomace. Food Chemistry 2000, 68, 81-85.

Miller N. J. (1998). Flavonoids and phenylpropanoids as contributors to the antioxidant activity of fruit juices in Flavonoids in health and disease etd Catherine Rice Evans and Lester Packer, Marcel Dekker Inc. chap 16, 387-403

Oszmianski J. et Lee C.Y. (1991). Enzymatic oxidation of phloretin glucoside in model system. Journal of Agricultural and Food Chemistry, 39,1050-1052.

Pulido R., Bravo L., Saura-Calixto F. Antioxidant activity of dietary polyphenols as determined by a modified ferric reducing/antioxidant power assay. J Agric Food Chem 2000, 48, 3396-3402.

Raa, J. and J. C. Overeem (1968). "Transformation reactions of phloridzin in the presence of apple leaf enzymes." Phytochemistry. 7: 721-731.

Ridgway T., G. Tucker, H. Wiseman. (1997). "Novel bioconversions for the production of designer antioxidant and colourant flavonoids using polyphenol oxidases." Biotechnology and Genetic Engineering Reviews 14: 165-190.

Ridgway, T., J. O'reilly, et al. (1996). "Potent antioxidant properties of novel apple-derived flavonoids with commercial potential as food additives." Biochemical Society Transactions 24(3): 391S.

Ridgway, T., G. Tucker (1999). Procedure for the partial purification of apple leaf polyphenol oxidase suitable for commercial application. Enzyme Microb. Technol. 25, 225-231

Ridgway, T. (1999). Colourants, antioxidants and phytoestrogens from apple. J. Chem. Technol. Biotechnol. 74, 377

Shoji T., KoboriM., Shinmoto H., Tanabe M., Tsusshida T., Progressive effects of phloridzin on melanogenesis in B16 melanoma cells. Biosci. Biotech. Biochem. 1997, 61, 12, 1963-1967.

## Revendications

**1.** Composés de formule générale (I) suivante :

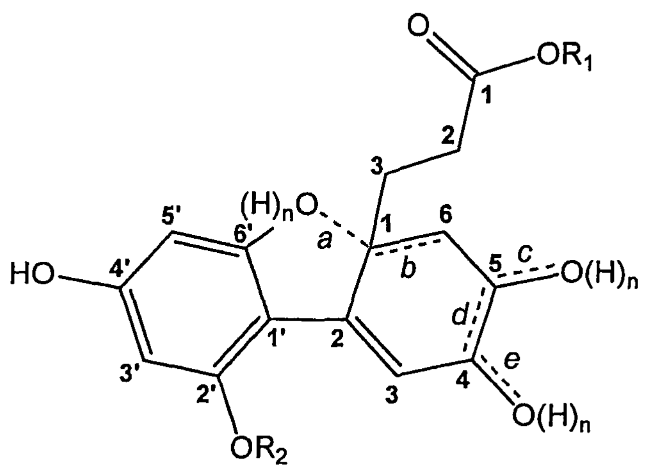

(I)

dans laquelle :

- n est égal à 0 ou 1,
- $R_1$ représente :
  - un atome d'hydrogène,
  - ou un groupe alkyle de 1 à 10 atomes de carbone,
- $R_2$ représente :
  - un atome d'hydrogène,
  - un groupe glucose, ou
  - un groupe xyloglucose,

et dans laquelle :

- lorsque n = 1, la liaison **a** n'existe pas, les liaisons **b** et **d** sont des doubles liaisons, et les liaisons **c** et **e** sont des liaisons simples, les composés correspondants étant désignés composés de formule **I-a,**
- lorsque n = 0, les liaisons **a**, **b** et **d** sont des liaisons simples, et les liaisons **c** et **e** sont des doubles liaisons, les composés correspondants étant désignés composés de formule I-**b**.

**2.** Composés selon la revendication 1, de formule I-**a** suivante :

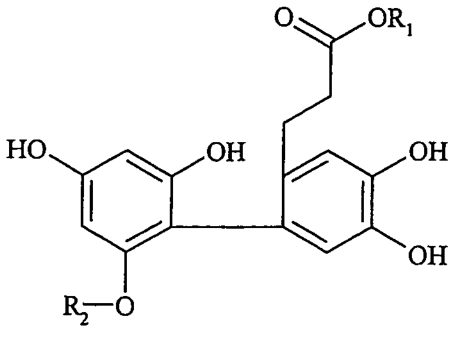

(I-**a**)

dans laquelle $R_1$ et $R_2$ sont tels que définis dans la revendication 1.

**3.** Composés selon la revendication 1, de formule I-**b** suivante :

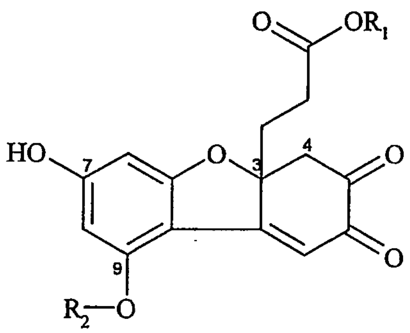

(I-**b**)

dans laquelle $R_1$ et $R_2$ sont tels que définis dans la revendication 1.

**4.** Composés selon l'une des revendications 1 à 3, de formule I-**a** ou **I-b** dans laquelle R1 représente H, un groupe méthyle $CH_3$, un groupe éthyle $CH_2$-$CH_3$, un groupe propyle $(CH_2)_2$-$CH_3$, un groupe isopropyle $CH(CH_3)$-$CH_3$, un groupe butyle $(CH_2)_3$-$CH_3$, un groupe hexyle $(CH_2)_5$-$CH_3$, ou un groupe octyle $(CH_2)_7$-$CH_3$., et $R_2$ est tel que défini dans la revendication 1.

**5.** Composés selon l'une des revendications 1 à 4, de formule I-**a-1** ou **I-b- 1** correspondant respectivement aux composés de formules I-**a** et I-**b** dans lesquelles $R_1$ représente H, et $R_2$ représente un glucose.

**6.** Procédé de préparation de composés l'une des revendications 1 à 5, **caractérisé en ce qu'**il comprend une étape de mise en présence de phloridzine, pure ou sous forme d'extrait brut de végétaux, en particulier, de pomme ou de pommier, ou d'une préparation contenant de la phloridzine, avec une polyphénol oxydase (PPO) d'origine végétale, fongique ou bactérienne, à activité crésolase et catécholase en présence d'oxygène de l'air ou d'une atmosphère enrichie en oxygène, suivie le cas échéant d'une étape de purification du composé ainsi obtenu de formule I-**a** ou **I-b** dans laquelle $R_1$=H, et $R_2$ est H, glucose, ou xyloglucose, et le cas échéant d'une étape de traitement de ce dernier composé avec un alcool de formule $R_1$-OH dans laquelle $R_1$ représente un groupe alkyle tel que défini ci-dessus, pour obtenir un composé de formule I-**a** ou **I-b** dans laquelle $R_1$ représente un groupe alkyle tel que défini ci-dessus, cette étape de traitement étant effectuée avant ou après l'étape de purification susmentionnée, et le cas

échéant, une étape de purification du composé de formule I-**a** ou I-**b** dans laquelle $R_1$ représente un groupe alkyle tel que défini ci-dessus obtenu lors de l'étape précédente.

**7.** Procédé selon la revendication 6, **caractérisée en ce que** la PPO utilisée est un extrait enzymatique de végétaux comestibles tels que la pomme et obtenu par :

- broyage des tissus apprêtés ou non dans un milieu permettant d'éviter toute oxydation,
- éventuellement tamisage de la pulpe obtenue lors de l'étape précédente de manière à éliminer les particules de taille supérieures à 50 $\mu$m et obtenir une suspension homogène,
- micro-filtration tangentielle de la suspension susmentionnée afin de concentrer la PPO,
- lavage de la suspension concentrée en cours de microfitration tangentielle par addition de tampon additionné d'acide ascorbique jusqu'à élimination des substrats de la PPO,
- lavage de la suspension concentrée en cours de microfitration tangentielle par addition de tampon non additionné d'acide ascorbique jusqu'à élimination de l'acide ascorbique,
- récupération du rétentat de micro-filtration tangentielle, centrifugation, refroidissement et, le cas échéant, congélation dans l'azote liquide.

**8.** Composition contenant le composé de formule I-**a** ou I-**b** selon la revendication 1, dans laquelle R1=H, et $R_2$ est H, glucose, ou xyloglucose, telle qu'obtenue par mise en présence d'un extrait végétal ayant une activité PPO selon la revendication 7, avec un extrait de phloridzine, de phlorétine ou de xyloglucoside de phlorétine, en présence d'oxygène de l'air ou d'une atmosphère enrichie en oxygène.

**9.** Composition contenant un composé de formule I-**a** ou I-**b** selon la revendication 1, dans laquelle $R_1$ représente un groupe alkyle tel que défini dans la revendication 1 ou 4, et $R_2$ est H, glucose, ou xyloglucose, telle qu'obtenue par traitement de la composition selon la revendication 8, avec un alcool de formule $R_1$-OH dans laquelle $R_1$ représente un groupe alkyle tel que défini dans la revendication 1 ou 4.

**10.** Composition selon la revendication 8 ou 9, **caractérisée en ce que** la proportion en poids des composés de formule I-**a** ou I-**b** dans ladite composition est d'environ 15% à environ 90%, notamment d'environ 50% à 80%.

**11.** Composition alimentaire **caractérisée en ce qu'**elle comprend au moins un composé de formule I-**a** ou I-**b** selon l'une des revendications 1 à 5, ou une composition selon l'une des revendications 8 à 10.

**12.** Utilisation d'au moins un composé de formule I-**b** selon l'une des revendications 1 et 3 à 5, ou d'une composition selon l'une des revendications 8 à 10, en tant que colorant de couleur jaune, notamment dans le cadre de l'alimentation humaine ou animale.

**13.** Composition pharmaceutique ou nutraceutique **caractérisée en ce qu'**elle comprend au moins un composé de formule I-**a** ou I-**b** selon l'une des revendications 1 à 5, ou d'une composition selon l'une des revendications 8 à 10, le cas échéant en association avec un véhicule pharmaceutiquement acceptable.

**14.** Utilisation d'au moins un composé de formule I-**a** ou I-**b** selon l'une des revendications 1 à 5, ou d'une composition selon l'une des revendications 8 à 10, pour la préparation d'un médicament à activité antioxydante et antiradicalaire, destiné notamment à la prévention ou au traitement du stress oxydatif et des pathologies liées au stress oxydatif, notamment des cancers.

**15.** Composition cosmétique **caractérisée en ce qu'**elle comprend au moins un composé de formule I-**a** ou I-**b** selon l'une des revendications 1 à 5, ou d'une composition selon l'une des revendications 8 à 10.

**16.** Méthode de traitement esthétique, **caractérisé en ce qu'**elle comprend l'application sur la peau d'un individu d'une composition cosmétique selon la revendication 15.

## Claims

**1.** Compounds of the following general formula (I):

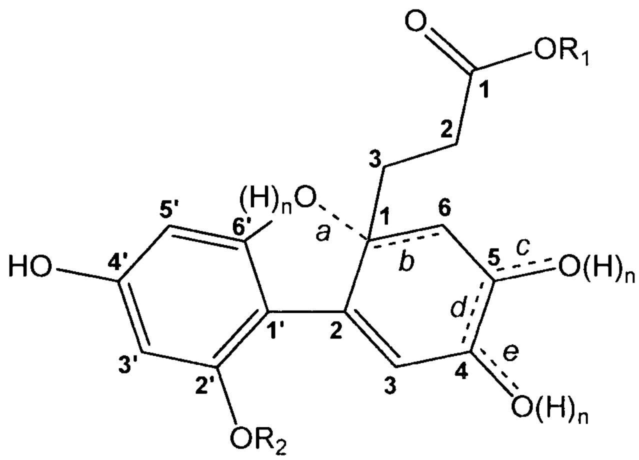

(I)

in which:

- n is equal to 0 or 1,
- $R_1$ represents:
  - a hydrogen atom,
  - or an alkyl group with 1 to 10 carbon atoms,
- $R_2$ represents:
  - a hydrogen atom,
  - a glucose group, or
  - a xyloglucose group,

and in which:

- when n = 1, bond **a** does not exist, bonds **b** and **d** are double bonds, and bonds **c** and **e** are simple bonds, corresponding compounds being designated as compounds of formula I-**a**,
- when n = 0, bonds **a, b** and **d** are single bonds, and bonds **c** and **e** are double bonds, corresponding compounds being designated as compounds of formula I-**b**.

**2.** Compounds according to claim 1, of the following formula (I-**a**):

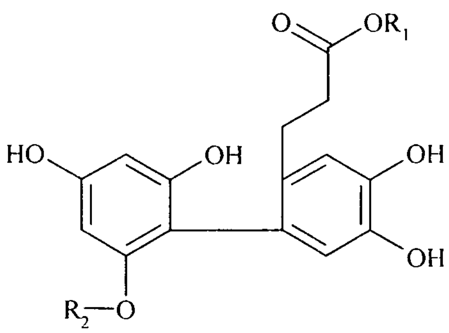

(I-**a**)

in which $R_1$ and $R_2$ are such as defined in claim 1.

**3.** Compounds according to claim 1, of the following formula (I-**b**):

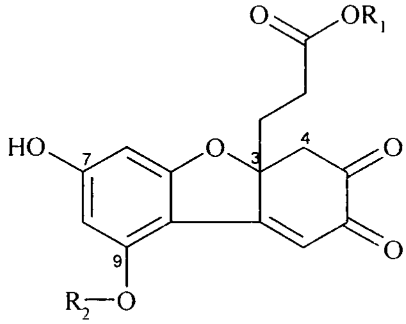

(I-**b**)

in which $R_1$ and $R_2$ are such as defined in claim 1

4. Compounds according to any one of claims 1 to 3, of formula I-**a** or I-**b** in which R1 represents H, a methyl group $CH_3$, an ethyl group $CH_2$-$CH_3$. a propyl group $(CH_2)_2$-$CH_3$, an isopropyl group $CH(CH_3)$-$CH_3$, a butyl group $(CH_2)_3$-$CH_3$, an hexyl group $(CH_2)_5$-$CH_3$, or an octyl group $(CH_2)_7$-$CH_3$, and $R_2$ is such as defined in claim 1.

5. Compounds according to one of claims 1 to 4, of formula I-**a-1** or I-**b-1** corresponding respectively to compounds of formulae I-**a** and I-**b** in which $R_1$ represents H, and $R_2$ represents a glucose.

6. Process for preparing compounds according to one of claims 1 to 5, **characterized in that** it includes a step of bringing into contact phloridzin, pure or in the form of crude extract of plants, in particular, of apple or of apple-tree, or of a preparation containing phloridzin, with a polyphenol oxidase (PPO) of plant, fungal or bacterial origin, with cresolase and catecholase activity in presence of oxygen from the air or in an oxygen-enriched atmosphere, followed if necessary by a step of purification of the compound thus obtained of formula I-**a** or I-**b** in which $R_1$=H, and $R_2$ is H, glucose, or xyloglucose, and if necessary a step of treatment of the latter compound with an alcohol of formula $R_1$-OH in which $R_1$ represents an alkyl group such as defined above, to obtain a compound of formula I-**a** or I-**b** in which $R_1$ represents an alkyl group such as defined above, this step of treatment taking place before or after the aforesaid purification step, and if necessary, a step of purification of the compound of formula I-**a** or I-**b** in which $R_1$ represents an alkyl group such as defined above obtained from the previous stage.

7. Process according to claim 6, **characterized in that** the used PPO is an enzymatic extract of edible plants such as apple and obtained by:

   - crushing of tissues prepared or not in a medium permitting to prevent oxidation,
   - optionally sieving of the pulp obtained from the previous step so to remove particles larger than 50 $\mu$m and to obtain a homogeneous suspension,
   - tangential microfiltration of the aforesaid suspension in order to concentrate the PPO,
   - washing of the concentrated suspension during its tangential microfiltration by addition of buffer doped with ascorbic acid until elimination of substrates from the PPO,
   - washing of the concentrated suspension during its tangential microfiltration by addition of buffer not doped with ascorbic acid until elimination of the ascorbic acid,
   - recovery of the tangential microfiltration retentate, centrifugation, cooling and, if necessary, freezing in liquid nitrogen.

8. Composition containing the compound of formula I-**a** or I-**b** according to claim 1, in which R1=H, and $R_2$ is H, glucose, or xyloglucose, such as obtained by bringing into contact a plant extract having a PPO activity according to claim 7, with an extract of phloridzin, phloretin or phloretin xyloglucoside, in presence of oxygen from the air or in an oxygen-enriched atmosphere.

9. Composition containing a compound of formula I-**a** or I-**b** according to claim 1, in which $R_1$ represents an alkyl group such as defined in claim 1 or 4, and $R_2$ is H, glucose, or xyloglucose, such as obtained by treatment of the composition according to claim 8, with an alcohol of formula $R_1$-OH in which $R_1$ represents an alkyl group such as defined in claim 1 or 4.

10. Composition according to claim 8 or 9, **characterized in that** the weight proportion of compounds of formula I-**a** or

I-**b** in said composition is from about 15% to about 90%, in particular from about 50% to 80%.

**11.** Dietary composition **characterized in that** it includes at least a compound of formula I-**a** or I-**b** according to one of claims 1 to 5, or a composition according to one of claims 8 to 10.

**12.** Use of at least one compound of formula I-**b** according to one of claims 1 and 3 to 5, or of a composition according to one of claims 8 to 10, as a colorant of yellow colour, in particular for the frame of human or animal alimentation.

**13.** Pharmaceutical or nutraceutical composition **characterized in that** it includes at least one compound of formula I-**a** or I-**b** according to one of claims 1 to 5, or of a composition according to one of claims 8 to 10, if necessary in combination with a pharmaceutical acceptable vehicle.

**14.** Use of at least one compound of formula I-**a** or I-**b** according to one of claims 1 to 5, or of a composition according to one of claims 8 to 10, for the preparation of a drug with antioxidizing and antiradical activity, intended in particular for prevention or treatment of oxidative stress and of pathologies linked to oxidative stress, in particular cancers.

**15.** Cosmetic composition **characterized in that** it includes at least one compound of formula I-**a** or I-**b** according to one of claims 1 to 5, or of a composition according to one of claims 8 to 10.

**16.** Method of aesthetic treatment, **characterized in that** it includes application on the skin of a person of a cosmetic composition according to claim 15.

**Patentansprüche**

**1.** Verbindungen nach der folgenden allgemeinen Formel:

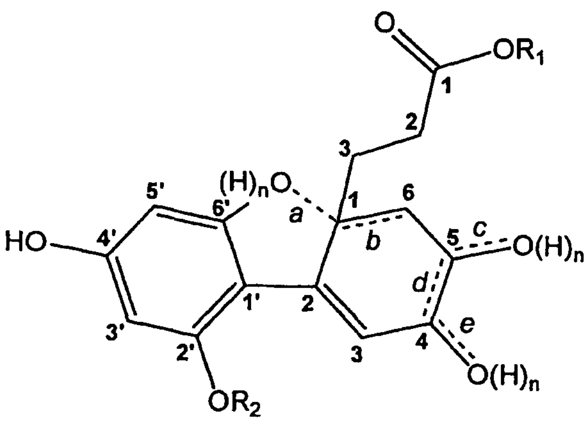

(I)

wobei:

- n gleich 0 oder 1 ist,
- $R_1$ für folgendes steht:

  - ein Wasserstoffatom,
  - oder eine Alkylgruppe mit 1 bis 10 Kohlenstoffatomen,

- $R_2$ für folgendes steht:

  - ein Wasserstoffatom,
  - eine Glucosegruppe, oder
  - eine Xyloglucosegruppe,

und wobei:

- wenn n = 1 ist, die Bindung a nicht besteht, die Bindungen **b** und **d** Doppelbindungen sind und die Bindungen **c** und **e** Einfachbindungen sind, wobei die entsprechenden Verbindungen als Verbindungen nach der Formel I-**a** bezeichnet werden,
- wenn n = 0 ist, die Bindungen **a, b** und **d** Einfachbindungen sind und die Bindungen **c** und **e** Doppelbindungen sind, wobei die entsprechenden Verbindungen als Verbindungen nach der Formel I-**b** bezeichnet werden.

**2.** Verbindungen nach Anspruch 1, nach der folgenden Formel I-**a**:

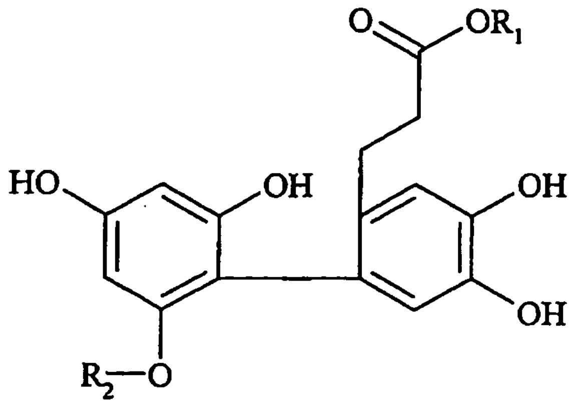

(I-a)

wobei $R_1$ und $R_2$ der Begriffsbestimmung in Anspruch 1 entsprechen.

**3.** Verbindungen nach Anspruch 1, nach der folgenden Formel I-**b**:

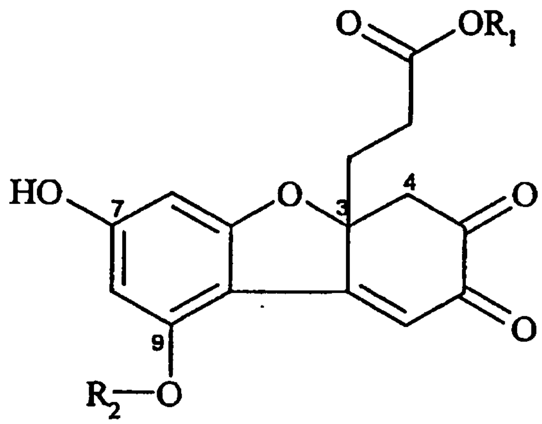

(I-b)

wobei $R_1$ und $R_2$ der Begriffsbestimmung in Anspruch 1 entsprechen.

**4.** Verbindungen nach einem der Ansprüche 1 bis 3, nach der Formel I-**a** oder I-**b**, wobei $R_1$ für H, eine Methylgruppe $CH_3$, eine Ethylgruppe $CH_2$-$CH_3$, eine Propylgruppe $(CH_2)_2$-$CH_3$, eine Isopropylgruppe $CH(CH_3)$-$CH_3$, eine Butylgruppe $(CH_2)_3$-$CH_3$, eine Hexylgruppe $(CH_2)_5$-$CH_3$ oder eine Octylgruppe $(CH_2)_7$-$CH_3$ steht und $R_2$ der Begriffsbestimmung in Anspruch 1 entspricht.

**5.** Verbindungen nach einem der Ansprüche 1 bis 4, nach der Formel I-**a-1** oder I-**b-1**, entsprechend den jeweiligen Verbindungen nach den Formeln I-**a** und I-**b**, wobei $R_1$ für H steht und $R_2$ für eine Glucoseeinheit steht.

**6.** Verfahren zur Herstellung von Verbindungen nach einem der Ansprüche 1 bis 5, **dadurch gekennzeichnet, dass** es einen Schritt des Zusammenbringens von Phloridzin, in Reinform oder in Form von Rohextrakt aus Pflanzen,

insbesondere aus Äpfeln oder Apfelbäumen, oder von einer Zubereitung, die Phloridzin enthält, mit einer Polyphenoloxidase (PPO), die aus Pflanzen, Pilzen, oder Bakterien stammt und in Gegenwart von Luftsauerstoff oder einer mit Sauerstoff angereicherten Atmosphäre eine Cresolase- und Catecholase-Aktivität besitzt, auf den gegebenenfalls ein Schritt folgt, in welchem die auf diese Weise erhaltene Verbindung nach der Formel I-**a** oder I-**b**, wobei $R_1$ = H und $R_2$ gleich H, Glucose oder Xyloglucose ist, aufgereinigt wird, und gegebenenfalls einen Schritt, in welchem die letztere Verbindung mit einem Alkohol der Formel $R_1$-OH, wobei $R_1$ für eine Alkylgruppe gemäß der obigen Begriffsbestimmung steht, behandelt wird, um eine Verbindung nach der Formel I-**a** oder I-**b**, wobei $R_1$ für eine Alkylgruppe gemäß der obigen Begriffsbestimmung steht, zu erhalten, wobei dieser Behandlungsschritt vor oder nach dem obengenannten Aufreinigungsschritt durchgeführt wird, und gegebenenfalls einen Schritt, in welchem die Verbindung nach der Formel I-**a** oder I-**b**, die im vorhergehenden Schritt erhalten wurde, wobei $R_1$ für eine Alkylgruppe gemäß der obigen Begriffsbestimmung steht, aufgereinigt wird, umfasst.

**7.** Verfahren nach Anspruch 6, **dadurch gekennzeichnet, dass** es sich bei der verwendeten PPO um einen enzymatischen' Extrakt von essbaren Pflanzen wie etwa dem Apfel handelt, der folgendermaßen erhalten wird:

- Zerkleinerung des aufbereiteten oder unbehandelten Gewebes in einem Milieu, das es ermöglicht, jegliche Oxidation zu vermeiden,
- möglicherweise Sieben des Breis, der im vorhergehenden Schritt erhalten wurde, sodass Partikel mit einer Größe von mehr als 50 $\mu$m entfernt werden und eine homogene Suspension erhalten wird,
- Querstrom-Mikrofiltration der obengenannten Suspension, um die PPO zu konzentrieren,
- Waschen der Suspension, die im Laufe der Querstrom-Mikrofiltration aufkonzentriert wurde, durch Zusatz eines mit Ascorbinsäure versetzten Puffers, bis das Substrat der PPO beseitigt ist,
- Waschen der Suspension, die im Laufe der Querstrom-Mikrofiltration aufkonzentriert wurde, durch Zusatz eines nicht mit Ascorbinsäure versetzten Puffers, bis die Ascorbinsäure beseitigt ist,
- Entnahme des Retentats der Querstrom-Mikrofiltration, Zentrifugation, Abkühlen und gegebenenfalls Einfrieren in flüssigem Stickstoff.

**8.** Zusammensetzung, die Verbindung nach der Formel I-**a** oder I-**b** nach Anspruch 1, wobei $R_1$ = H und $R_2$ gleich H, Glucose oder Xyloglucose ist, enthaltend und derart beschaffen, wie sie durch das Zusammenbringen eines Pflanzenextrakts der eine PPO-Aktivität gemäß Anspruch 7 hat, mit einem Extrakt von Phloridzin, Phloretin oder Phloretin-Xyloglucosid in Gegenwart von Luftquerstoff oder einer mit Sauerstoff angereicherten Atmosphäre erhalten wird.

**9.** Zusammensetzung, eine Verbindung nach der Formel I-**a** oder I-**b** nach Anspruch 1, wobei $R_1$ für eine Alkylgruppe gemäß der Begriffsbestimmung in Anspruch 1 oder 4 steht und $R_2$ gleich H, Glucose oder Xyloglucose ist, enthaltend und derart beschaffen, wie sie durch die Behandlung der Zusammensetzung gemäß Anspruch 8 mit einem Alkohol der Formel $R_1$-OH, wobei $R_1$ für eine Alkylgruppe gemäß der Begriffsbestimmung in Anspruch 1 oder 4 steht, erhalten wird.

**10.** Zusammensetzung nach Anspruch 8 oder 9, **dadurch gekennzeichnet, dass** der Gewichtsanteil der Verbindungen nach der Formel I-**a** oder I-**b** in der Zusammensetzung ungefähr 15 % bis ungefähr 90 %, insbesondere ungefähr 50 % bis 80 % beträgt.

**11.** Nahrungsmittelzusammensetzung, **dadurch gekennzeichnet, dass** sie mindestens eine Verbindung nach der Formel I-**a** oder I-**b** nach einem der Ansprüche 1 bis 5 oder eine Zusammensetzung nach einem der Ansprüche 8 bis 10 umfasst.

**12.** Verwendung mindestens einer Verbindung nach der Formel I-**b** nach einem der Ansprüche 1 und 3 bis 5 oder einer Zusammensetzung nach einem der Ansprüche 8 bis 10 als gelber Farbstoff, insbesondere im Rahmen der Ernährung von Menschen oder Tieren.

**13.** Pharmazeutische oder nutrazeutische Zusammensetzung, **dadurch gekennzeichnet, dass** sie mindestens eine Verbindung nach der Formel I-**a** oder I-**b** nach einem der Ansprüche 1 bis 5 oder eine Zusammensetzung nach einem der Ansprüche 8 bis 10, gegebenenfalls in Verbindung mit einem pharmazeutisch unbedenklichen Träger umfasst.

**14.** Verwendung mindestens einer Verbindung nach der Formel I-**a** oder I-**b** nach einem der Ansprüche 1 bis 5 oder einer Zusammensetzung nach einem der Ansprüche 8 bis 10 zur Herstellung eines Arzneimittels mit antioxidativer und radikalbekämpfender Wirkung, insbesondere zur Vorbeugung oder zur Behandlung von oxidativem Stresses

und von Erkrankungen, die mit oxidativem Stress verbunden sind, insbesondere von Krebserkrankungen, bestimmt.

**15.** Kosmetische Zusammensetzung, **dadurch gekennzeichnet, dass** sie mindestens eine Verbindung nach der Formel I-**a** oder I-**b** nach einem der Ansprüche 1 bis 5 oder eine Zusammensetzung nach einem der Ansprüche 8 bis 10 umfasst.

**16.** Verfahren zur ästhetischen Behandlung, **dadurch gekennzeichnet, dass** es das Aufbringen einer kosmetischen Zusammensetzung nach Anspruch 15 auf die Haut eines Individuums umfasst.

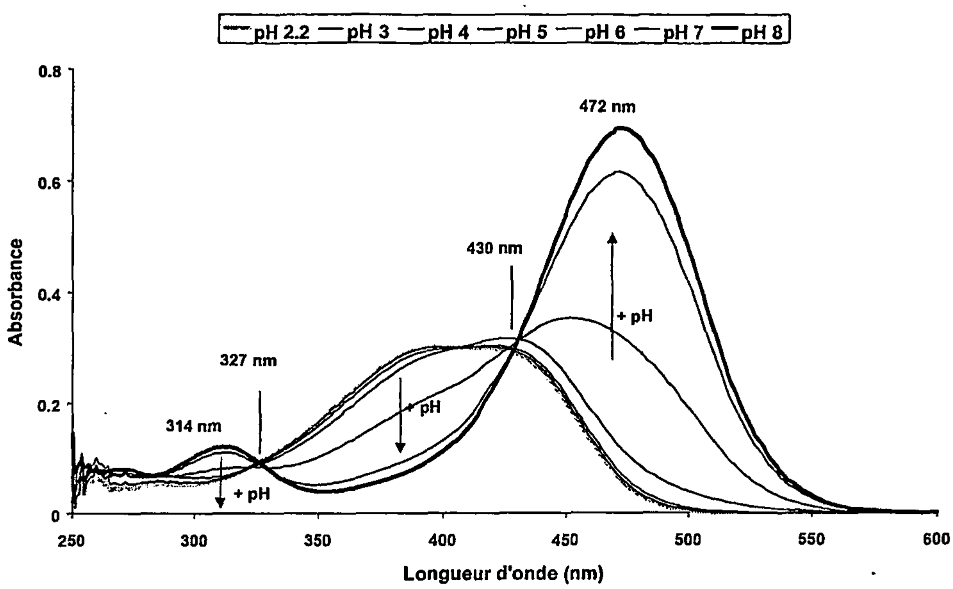


Figure 1 - Effet du pH sur le spectre UV-visible du colorant POP en absorbance.

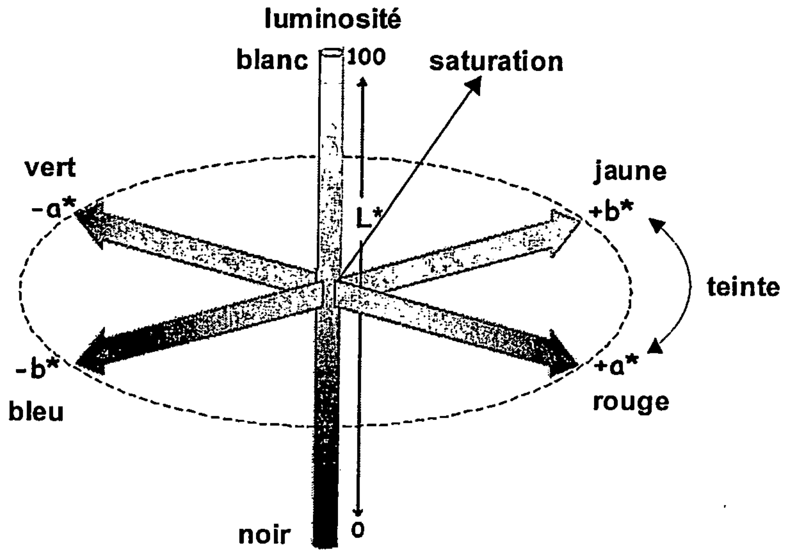


figure 2 : Espace colorimétrique CIE L*a*b*

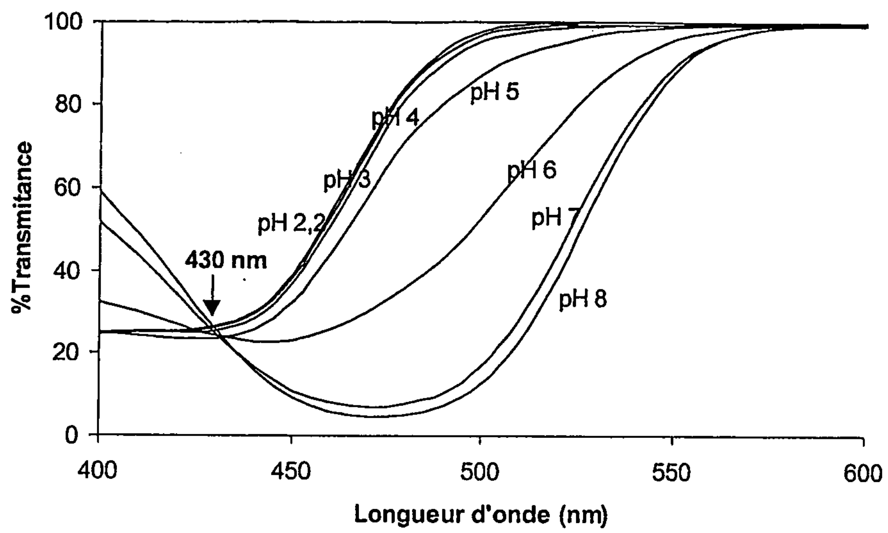


figure 3 : Effet du pH sur le spectre visible du colorant POP en % de transmittance.

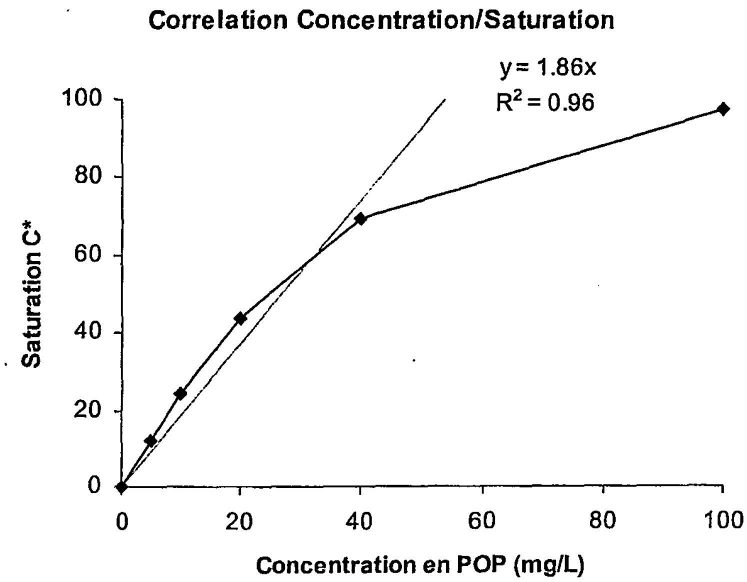


figure 4 : Corrélation de la saturation avec la concentration de colorant POP.

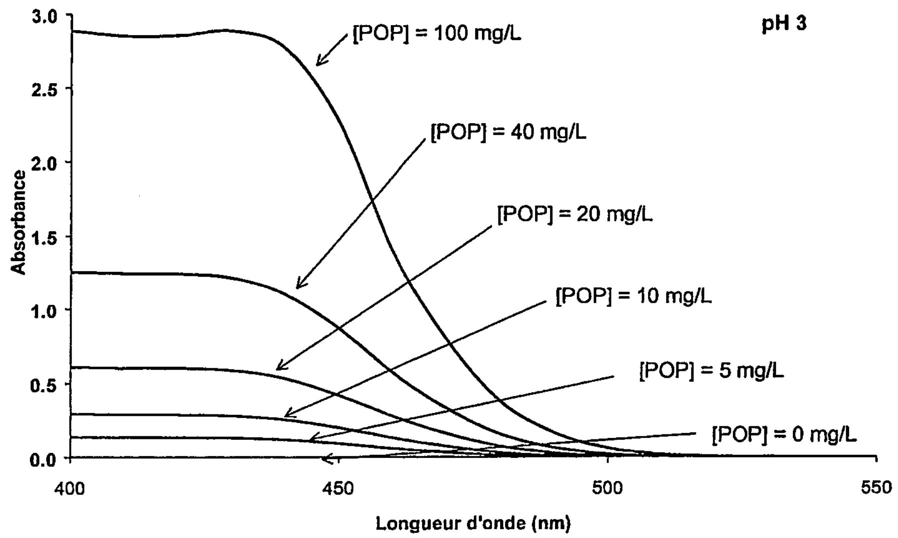


figure 5 : Effet de la concentration en colorant POP sur l'absorbance dans le visible.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0612814 A
- WO 0191585 A2


### Littérature non-brevet citée dans la description


- **Brand-Williams W. ; Cuvelier M.E. ; Berser C.** Use of a Free Radical Method to Evaluate Antioxidant Activity. *Lebensin.-Wiss. u.-Tecfinol.,* 1995, vol. 28, 25-30
- **Frerichs H. ; Ball E.G.** Studies on the metabolism of Adipose Tissue. XVI. Inhibition by phlorizin and phloretin of the insulin-stimulated uptake of glucose. *Biochemistry,* 1964, vol. 3 (7), 981-985
- **Lea, A. G. H.** Farb- und gerbstoffe in englischen mostapfeln. *Flüssiges Obst,* 1984, vol. 8, 356-361
- **Lu Y. ; Foo L. Y.** Antioxidant and radical scavenging activities of polyphenols from apple pomace. *Food Chemistry,* 2000, vol. 68, 81-85
- Flavonoids and phenylpropanoids as contributors to the antioxidant activity of fruit juices. **Miller N. J.** Flavonoids in health and disease etd Catherine Rice Evans and Lester Packer. Marcel Dekker Inc, 1998, 387-403
- **Oszmianski J. ; Lee C.Y.** Enzymatic oxidation of phloretin glucoside in model system. *Journal of Agricultural and Food Chemistry,* 1991, vol. 39, 1050-1052
- **Pulido R. ; Bravo L. ; Saura-Calixto F.** Antioxidant activity of dietary polyphenols as determined by a modified ferric reducing/antioxidant power assay. *J Agric Food Chem,* 2000, vol. 48, 3396-3402
- **Raa, J. ; J. C. Overeem.** Transformation reactions of phloridzin in the presence of apple leaf enzymes. *Phytochemistry,* 1968, vol. 7, 721-731
- **Ridgway T. ; G. Tucker ; H. Wiseman.** Novel bioconversions for the production of designer antioxidant and colourant flavonoids using polyphenol oxidases. *Biotechnology and Genetic Engineering Reviews,* 1997, vol. 14, 165-190
- **Ridgway, T. ; J. O'reilly et al.** Potent antioxidant properties of novel apple-derived flavonoids with commercial potential as food additives. *Biochemical Society Transactions,* 1996, vol. 24 (3), 391S
- **Ridgway, T. ; G. Tucker.** Procedure for the partial purification of apple leaf polyphenol oxidase suitable for commercial application. *Enzyme Microb. Technol.,* 1999, vol. 25, 225-231
- **Ridgway, T.** Colourants, antioxidants and phytoestrogens from apple. *J. Chem. Technol. Biotechnol.,* 1999, vol. 74, 377
- **Shoji T. ; KoboriM. ; Shinmoto H. ; Tanabe M. ; Tsusshida T.** Progressive effects of phloridzin on melanogenesis in B16 melanoma cells. *Biosci. Biotech. Biochem,* 1997, vol. 61 (12), 1963-1967